# EUROPEAN PATENT APPLICATION

(11) **EP 1 972 354 A1**
(43) Date of publication of application: **24.09.2008**
(21) Application number: 08250546.2
(22) Date of filing: 15.02.2008
(51) Int. Cl.: A61M 1/36, A61M 1/38, A61M 1/02

(54) **Methods and systems for preparing blood products**

(30) Priority: 20.03.2007 US 688694
(71) Applicant: CaridianBCT Biotechnologies, LLC, Lakewood CO 80215 (US)
(72) Inventor: Hlavinka, Dennis J., Arvada, CO 80007 (US)
(74) Representative: Roberts, Mark Peter

(57) **Abstract**

The present invention is related to pre-connected disposable sets (70,72,74) including a pre-connected bag (62,64,66,32,34,36) containing a photosensitizer (75) for use in a blood separation and pathogen inactivation procedure.

## Description

Whole blood collected from healthy donors is routinely separated into components: platelets, plasma and red blood cells. One or more of the separated components are used for later administration (transfusion) to a patient who may be in need of the particular component. For example, red blood cells may be administered to a patient to replace blood loss or to treat patients with chronic anemia. Plasma may be administered to treat clotting factor deficiencies. Platelets are commonly administered as a therapy to cancer patients whose ability to generate platelets has been compromised by chemotherapy.

There are traditionally two ways to obtain these blood components. One way is to collect whole blood from donors/patients and separate it into components manually at some time period after the whole blood collection.

Another way to separate whole blood into components is by using automated cell-separation devices. Apheresis machines are devices which automatically separates whole blood into components, and returns any uncollected blood components back to the donor during the collection procedure.

An alternative to manual processing of whole blood as described above is the automatic processing of whole blood using an automated device.

Whether whole blood is separated manually, by apheresis, or by automatic processing, before transfusion to a patient, the separated blood components may be subjected to an additional treatment to ensure the safety of the blood component intended for transfusion. Specifically, the collected blood components are treated to remove or otherwise inactivate virus and/or bacteria ("pathogens") which may reside in the particular blood component. Many pathogen inactivation methods involve combining the blood component to be inactivated with a photosensitizer compound, which, when stimulated by light, acts on the pathogen, destroying its ability to replicate.

The steps of separating blood into components and pathogen reducing the components are not typically done together as part of one procedure. At some point in time after the blood is separated into components, the photosensitizer compound and blood component to be inactivated are typically added to a separate illumination bag to be exposed to light. Such procedure requires additional bags and additional sterile connections of bags, thus increasing the possibility for contamination during the multiple connections.

It would therefore be desirable to provide disposable sets for separating blood into components which includes a photosensitizer compound prepackaged within the sets to allow for easy preparation of such treatment-ready blood products. It is to the simplification of the blood separation and pathogen reduction process that the present invention is directed.

The invention relates to a pre-connected disposable set and a method of inactivating pathogens. The invention provides for a pre-connected bag containing a photosensitizer.

In one embodiment, the invention provides a pre-connected disposable set for blood separation and pathogen reduction comprising a separation vessel and at least one product bag pre-connected via a product collection conduit to the separation vessel. The at least one product bag may contain a photosensitizer, or there may be a second bag containing photosensitizer which is fluidly connected to the at least one product bag. There may also be an additional bag to collect whole blood fluidly connected to the separation vessel. Any one, some or all of the bags may be covered by a removable opaque overwrap.

Also claimed is a method of using the above disposable set.

In another embodiment, the invention provides a method of pathogen inactivating blood components which may contain pathogens comprising: providing a first separated blood component; flowing the first separated blood component into a pre-connected product bag containing a photosensitizer; wherein the product bag is covered by a removable opaque overwrap; removing the removable opaque overwrap; and illuminating the first separated blood component and photosensitizer for a time sufficient to inactivate any pathogens which may be present in the blood component but less than a time which would cause damage to the blood component.

In a further embodiment, the invention provides a method of pathogen inactivating blood components which may contain pathogens comprising: providing a first separated blood component; flowing the first separated blood component into a pre-connected product bag; flowing a photosensitizer from a bag pre-connected to the product bag into the product bag; and illuminating the product bag for a time sufficient to inactivate any pathogens which may be present in the blood component but less than a time which would cause damage to the blood component.

The invention will now be further described, by way of example only, with reference to the accompanying drawings, in which:-
FIG. 1 is a schematic view of a set of separation and collection bags designed for cooperating with an automated apheresis apparatus;
FIG. 2 is a schematic view of another embodiment of the set of separation and collection bags shown in FIG. 1;
FIG. 3 is a schematic view of a set of separation and collection bags designed for cooperating with an automated whole blood separation apparatus;
FIG.4 is a schematic view of another embodiment of the set of separation and collection bags shown in FIG. 3;
FIG. 5 is a schematic view of another set of separation and collection bags designed for cooperating with another automated whole blood separation apparatus;
FIG. 6 is a schematic view of another embodiment of the set of separation and collection bags shown in FIG. 5; and
FIG. 7 shows an embodiment of this invention for treating the blood component to be inactivated with light.

A "photosensitizer" useful in this invention is defined as any compound which absorbs radiation at one or more defined wavelengths and subsequently utilizes the absorbed energy to carry out a chemical process.

Many photosensitizers may be used in this invention. Porphyrins, psoralens, quinacrines, and dyes such as methylene blue may all be used with this invention.

Endogenous photosensitizers may also be used. The term "endogenous" means naturally found in a human or mammalian body, either as a result of synthesis by the body or because of ingestion as an essential foodstuff (e.g. vitamins) or formation of metabolites and/or byproducts in vivo. When endogenous photosensitizers are used, particularly when such photosensitizers are not inherently toxic or do not yield toxic photoproducts after photoradiation, no removal or purification step is required after decontamination, and the decontaminated product can be directly returned to a patient's body or administered to a patient in need of its therapeutic effect.

Examples of such endogenous photosensitizers which may be used in this invention are alloxazines such as 7,8-dimethyl-10-ribityl isoalloxazine (riboflavin), 7,8,10-trimethylisoalloxazine (lumiflavin), 7,8-dimethylalloxazine (lumichrome), isoalloxazine-adenine dinucleotide (flavin adenine dinucleotide [FAD]) and alloxazine mononucleotide (also known as flavin mononucleotide [FMN] and riboflavin-5-phosphate). The term "alloxazine" includes isoalloxazines.

Use of endogenous isoalloxazines as photosensitizers to inactivate blood and blood components are described in United States Patent Nos. 6, 258,577 and 6,277,337 both issued to Goodrich et al., and herein incorporated by reference to the amount not inconsistent.

The amount of photosensitizer to be mixed with the blood and blood components to be inactivated will be an amount sufficient to adequately inactivate any pathogenic nucleic acids which may be present in the fluid, but less than a toxic (to the desired components) or insoluble amount. If riboflavin is used as the photosensitizer, it may be added to the fluid at a final concentration of between about 50-500 µM. Pathogenic nucleic acid includes any undesirable nucleic acid such as nucleic acid contained in white blood cells, bacteria or viruses. Nucleic acids include either deoxyribonucleic acid (DNA), ribonucleic acid (RNA) or both.

The blood or blood components to which the photosensitizer has been added is exposed to light of the appropriate wavelength to activate the photosensitizer and to cause substantially permanent damage to the pathogenic nucleic acids.

### Apheresis disposable set

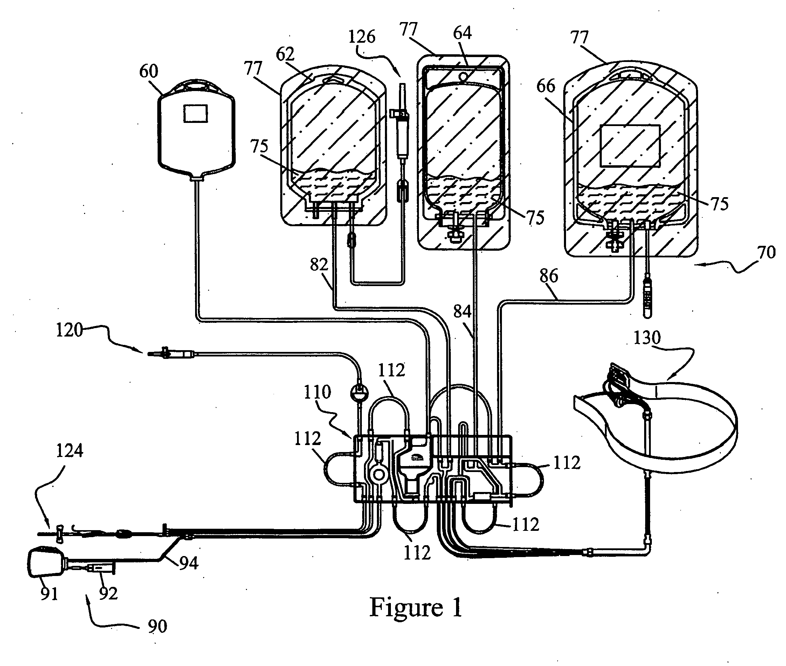

FIG. 1 shows a typical disposable set **70** for use with an apheresis machine. One such machine which may be used is the TRIMA® apheresis machine, available from Gambro BCT, Inc. (Lakewood, CO, USA).

It should be noted that in the Figures, like elements are denoted using like numerals.

The disposable set **70** may include a separation vessel **130** for separating blood into various blood components. The set **70** may also include a fluid flow cassette **110** having peristaltic pump loops **112,** a receive and return line **124** for receiving and returning blood and blood components to and from a donor, an anticoagulant line **120** for adding anticoagulant to the blood withdrawn from the donor, and a gas line **80** for receiving, into bag **60,** gas purged from the system. The tubing set **70** and the separation vessel **130** are in this embodiment interconnected to create a closed disposable for a single use.

Various collection lines and bags for receiving separated blood components may also be part of the disposable set **70.**

Red blood cell collection line or conduit **82** receives separated red blood cells from separation vessel **130** and leads to red blood cell product bag **62.** A spike connection **126** is available to add storage solution (as but one example) to the red blood cells in product bag **62** if desired.

Plasma collection line or conduit **84** receives separated plasma from separation vessel 130 and leads to plasma product bag **64.**

Platelet collection line **86** receives collected platelets from the separation vessel and leads to platelet product bag **66.**

A sample set **90** may also be part of the disposable set **70.** The sample set **90** includes line or conduit **94** through which a blood sample fluidly flows to sample bag **91.** A blood sample may then be retrieved through sample device **92.**

A solution containing the photosensitizer **75** may be placed in one, two or all of the product bags **62, 64** and **66** during manufacture and assembly of the bag set. The separated blood components will flow from the separation vessel **130** into the product bags which may already contain the photosensitizer **75.**

To prevent degradation of the photoactive material in the product bag(s) before use, as well as to prevent evaporation of the photoactive solution during storage of the disposable set, the product bags may be contained within a removable opaque overwrap **77** which would prevent ambient light from activating the photoactive material before the blood product is added. The particular composition of the light filtering material will vary according to the light sensitivity spectrum of the photoactive material being used. If riboflavin is the photosensitizer being used, the overwrap **77** is preferably made of foil. In general, the overwrap is opaque at least at the wavelength(s) for which the photosensitizer is activated.

By having the photosensitizer already in one or more of the product bags or in a second bag preconnected to one or more of the product bags, the steps of connecting an illumination bag to each product bag, connecting a bag containing the photosensitizer to the illumination bag, flowing the product to be inactivated from the product bag into the illumination bag, and flowing the photosensitizer solution into the product in the illumination bag are all eliminated, reducing the likelihood of contamination during the multiple connections.

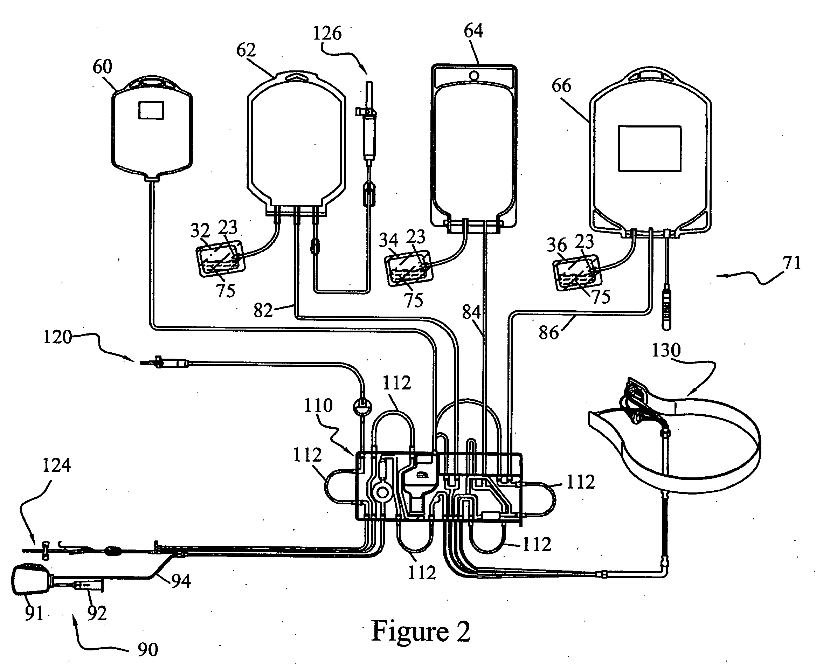

In another embodiment of a blood separation and pathogen reduction disposable set 71 shown in FIG. 2, bags **32, 34** and **36** containing riboflavin solution **75** may be respectively fluidly connected to each product bag **62, 64** and **66** respectively by a conduit. The bags containing the riboflavin solution may be wrapped in a protective foil overwrap to prevent degradation of the photosensitizer by ambient light and prevent evaporation of the solution through the bag during storage of the disposable set.

After the product is collected in each bag, the frangible connectors **23** located in each riboflavin solution bag **32, 34** and **36** could be broken to allow the riboflavin solution to drain into the product bag. The product bag could then be illuminated. Although three product bags **62, 64, 66** and three photosensitizer bags **75** are illustrated, the invention can be carried out with just one or two product and photosensitizer bags or indeed with four or more such bags.

In another embodiment, a bag containing the photoactive material may be attached to the disposable set **70** through spike port **120** (as but one example), flowed through the fluid flow cassette **110,** the separation vessel **130,** and into the product bag(s) **62, 64, 66** after the product(s) have been collected.

Although as shown in FIGS. 1 and 2 there are three product bags to collect platelets, plasma and red blood cells, it should be noted that only one product bag could be pre-connected to the separation vessel via a product conduit, to collect only one type of blood component. There could also be two product bags pre-connected to the separation vessel via product conduits to collect two types of blood components.

### Automatic whole blood processing disposable set

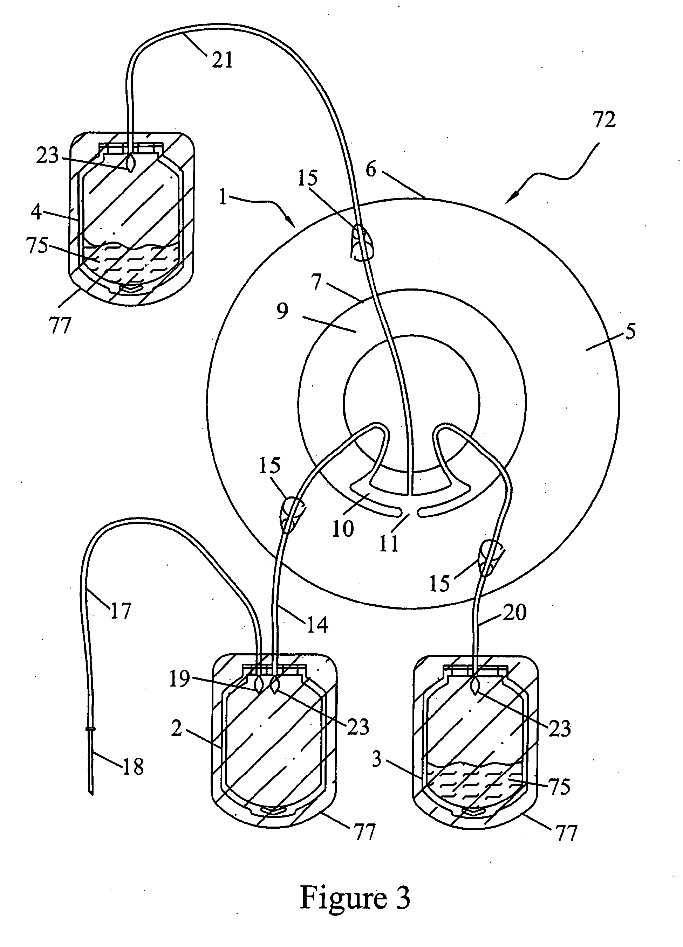

FIG. 3 shows a disposable bag set **72** for use in the separation and collection of previously collected whole blood into a plasma product, a platelet product and a red blood cell product using an automated whole blood separation device. The bag set can be used with an automated whole blood separation device, using a machine such as the Atreus® machine, manufactured by Gambro BCT, Inc. (Lakewood, CO, USA.) and described in PCT/US2006/031492. This set **72** comprises a separation vessel which may be a bag **1** for separating whole blood into various blood components, and three product collection bags **2, 3** and **4.**

The separation bag **1** comprises an annular separation chamber **5** having a substantially circular outer edge **6** and an inner circular edge **7.** The separation bag **1** further comprises a semi-flexible disk-shaped connecting element **9** that is connected to the inner edge **7** of the annular chamber **5.** The disk-shaped connecting element **9** comprises a distribution channel **10** embedded therein, which communicates through a passage **11** with the annular chamber **5.** The distribution channel **10** substantially extends along an arc of the circle.

The first product bag **2** has two purposes and is successively used as both a whole blood collection bag and as a red blood cell component bag. The first product bag is intended for initially receiving a volume of whole blood from a donor (usually about 450 ml) before the separation process, and subsequently the red blood cell component during the separation process. The first product bag **2** is flat and substantially rectangular. It is connected to the separation bag **1** by a first transfer tube **14,** fitted with a clamp **15.** The first transfer tube **14** has a first end connected to the upper edge of the first product bag **2** and a second end connected to a first end of the distribution channel **10.**

Anticoagulant is typically added to the first product bag **2** before whole blood is added. Typically about 63 ml of anticoagulant solution is added to a whole blood donation of about 450 ml. A frangible pin **23** removable from within the first product bag **2** prevents the anticoagulant from flowing out of the first product bag **2** into the separation bag **1** through the first transfer tube **14.** The frangible pin **23** could also be located within transfer tube **14.**

Whole blood collection tube **17** is connected at one end to the upper edge of the first product bag **2** and comprises, at the other end, a needle **18.** A frangible pin **19** removable from within the first product bag **2** plugs the downstream end of the collection tube **17** and prevents the anticoagulant solution from flowing out of the first product bag **2** through the collection tube **17.**

The second product bag **3** is intended for receiving a plasma component. It is flat and substantially rectangular. It is connected by a second transfer tube **20** to the separation bag **1.** The second transfer tube **20,** which is fitted with a clamp **15,** has a first end connected to the upper edge of the second product bag **3** and a second end connected to a second end of the distribution channel **10.**

The third product bag **4** is intended for receiving a platelet cell component. It is also flat and substantially rectangular. It is connected by a third transfer tube **21** to the separation bag **1.** The third transfer tube **21** has a first end connected to the upper edge of the third product bag **4** and a second end that is connected to the distribution channel **10** so as to face the passage **11** between the distribution channel **10** and the separation chamber **5.** The third transfer tube **21** is also fitted with a clamp **15.**

If leukoreduction of the red blood cell product is desired, the first product bag **2** may be connected to a leukoreduction filter (not shown). The filter could be located in line with tubing **14.**

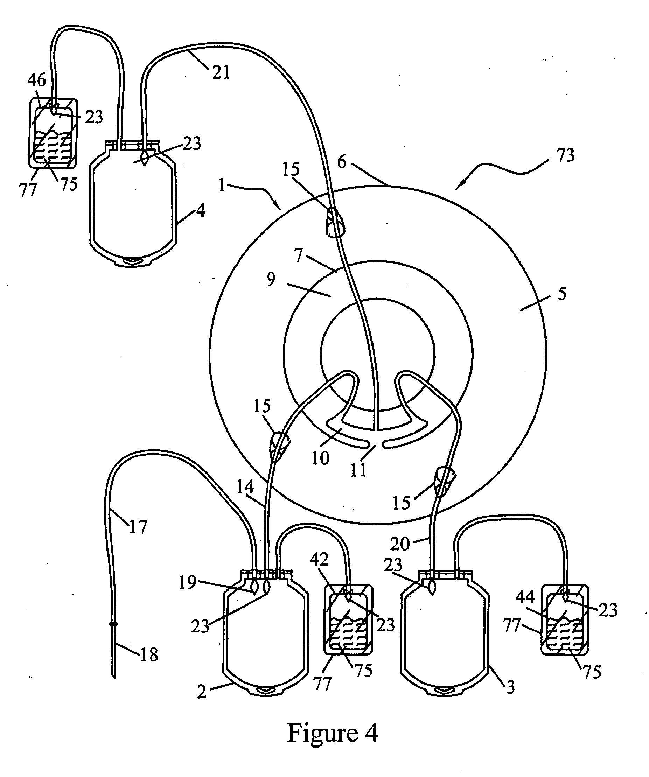

A solution containing the photosensitizer **75** may be placed in at least product bags **3** and **4** during manufacture and assembly of the bag set. The separated blood components would flow from the separation vessel **1 into** the product bags **3, 4** which would already contain the photosensitizer **75.** Although not shown in FIG. 3 (but see FIG. 4), a second bag containing photosensitizer solution can be fluidly connected by a conduit to bag **2.** After the separated red blood cell component is returned to bag **2,** the photosensitizer solution can be added. Frangible pins **23** would prevent the flow of photosensitizer **75** from the product bags **2, 3, 4** into the separation bag **1.**

To prevent degradation of the photoactive material **75** in the product bags **2, 3** and **4** before use, the product bags may be contained within a removable opaque overwrap **77** which prevents ambient light from activating the photoactive material as well as preventing evaporation of the photoactive solution during storage of the tubing set. The particular composition of the light filtering material will vary according to the light sensitivity spectrum of the photoactive material being used. If riboflavin is the photosensitizer being used, the overwrap **77** is preferably made of foil.

By having the photosensitizer either in the product bags or in a second bag preconnected to the product bags, the steps of connecting an illumination bag to each product bag, connecting a bag containing the photochemical to the illumination bag, flowing the product into the illumination bag, and flowing the photochemical solution into the product in the illumination bag are all eliminated, reducing the likelihood of contamination during the multiple connections.

In another embodiment of a blood separation and pathogen reduction set **73** shown in FIG. 4, bags **42, 44** and **46** containing riboflavin solution **75** can be fluidly connected to each product bag **2, 3** and **4** respectively. The bags containing the riboflavin solution **75** can be wrapped in a protective foil overwrap **77** to prevent degradation of the photosensitizer by ambient light and prevent evaporation of the solution through the bag.

After the product is collected in each bag, the frangible connectors **23** located in each riboflavin solution bag **42, 44** and **46** can be broken to allow the riboflavin solution to drain into the product bag/s. The product bag/s could then be illuminated.

In another embodiment, an additional bag (not shown) containing the photosensitizer may also be attached to the separation bag **1.** The photosensitizer may be metered from the additional bag through the separation bag into the product bags after the separated blood components have been collected in the product bags. Alternatively, the photosensitizer may be metered into the separation bag before the separation process. The separated blood products would then already contain photosensitizer when flowed into the product bags.

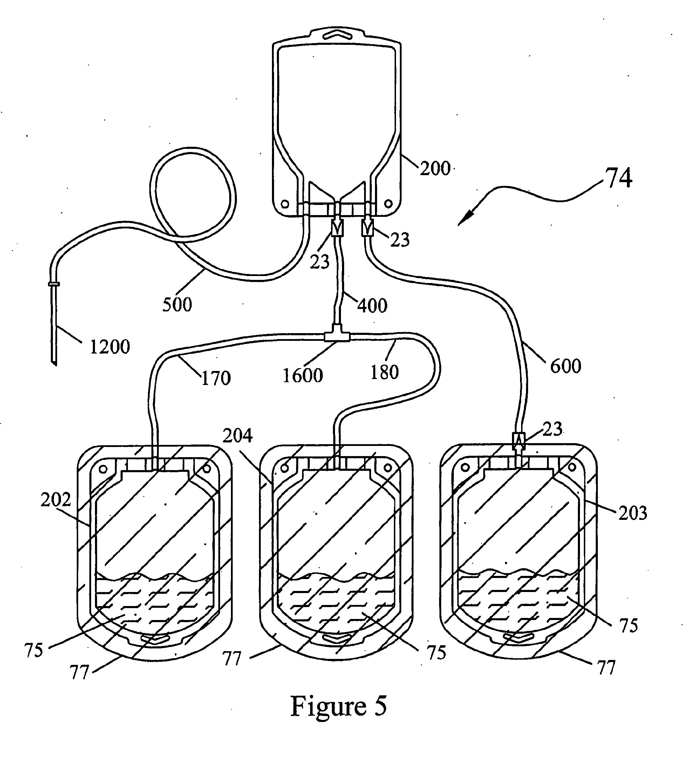

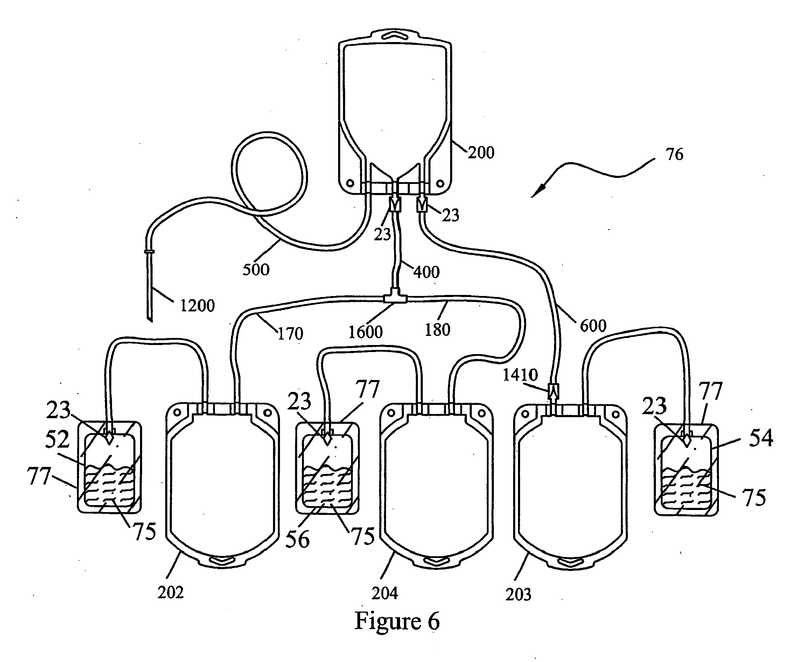

Although as shown in FIGS. 5 and 6 there are three product bags to collect platelets, plasma and red blood cells, it should be noted that only one product bag could be pre-connected to the separation vessel via a product conduit, to collect only one type of blood component. There could also be two product bags pre-connected to the separation vessel via product conduits to collect two types of blood components.

### Self-balancing bag set

Another disposable bag set **74,** which may be used with another automated whole blood separation device which is self balancing, is shown in FIG. 5. This disposable set may be used with another automated whole blood separation machine which is self balancing, and described in PCT/US2006/021674. This bag set comprises a flexible separation bag **200** and three flexible blood product bags **202, 203, 204** connected thereto.

The separation bag **200** has at least two purposes, and is successively used as both a whole blood collection bag and as a separation bag. It is intended for initially receiving a discrete volume of whole blood from a donor (usually about 450 ml) and to be used later as a separation chamber in a separation apparatus. The separation bag 200 is flat and generally rectangular. It is made of two rectangular sheets of plastic material that are welded together so as to define therebetween an interior space having a main rectangular portion connected to a triangular top downstream portion. A first tube **400** is connected to the tip of the triangular portion, and second and third tubes **500, 600** are connected to both lateral edges of the triangular portion, respectively. The proximal ends of the three tubes **400, 500, 600** are embedded between the two sheets of plastic material so as to be parallel.

The separation bag **200** may initially contain a volume of anti-coagulant solution, and the first and third tubes **400, 600** are fitted at their proximal ends with frangible connectors **23** respectively, blocking the flow of fluid therethrough.

The second tube **500** is a collection tube having a needle **1200** connected to its distal end. At the beginning of a blood donation, the needle **1200** is inserted in the vein of a donor and blood flows into the collection (separation) bag **200.** After a desired volume of blood has been collected in the collection (separation) bag **200,** the collection tube **500** is sealed and cut.

The first product bag **202** is intended for receiving a separated plasma component. It is flat and substantially rectangular. It is connected to the distal end of the first tube **400.**

The second product bag **203** is intended for receiving a separated red blood cell component. It is flat and substantially rectangular. It is connected to the distal end of the third tube **600.** The red blood cell collection bag **203** may contain a volume of storage solution for red blood cells, and the third tube **600** is fitted at its distal end with a frangible connector **23** blocking a liquid flow therethrough. An in-line leukoreduction filter (not shown) may also be attached in tubing line **600** if leukoreduction is desired.

The third product bag **204** is intended to receive a platelet component, and a T-shaped three-way connector **1600** having its leg connected by the first tube **400** to the separation bag **200,** a first arm connected by a fourth tube **170** to the collected plasma bag **202,** and a second arm connected by a fifth tube **180** to the collected platelet bag **204.** Like the plasma and red blood cell bags **202, 203,** the platelet bag **204** is flat and substantially rectangular.

The photosensitizer **75** may be placed in each of the product bags **202, 203** and **204** during manufacture and assembly of the bag set. The separated blood components may then flow from the separation vessel **200** into the storage bags which will already contain the photochemical.

In another embodiment of an automated whole blood separation device **76** shown in FIG. 6, bags **52, 54, 56** containing riboflavin solution **75** could be fluidly connected to each product bag **202, 203** and **204** respectively. The bags containing the riboflavin solution **75** can be wrapped in a protective foil overwrap **77** to prevent degradation of the photosensitizer by ambient light and prevent evaporation of the solution through the bag.

After the product is collected in each bag, the frangible connectors **23** located in each riboflavin solution bag **52, 54, 56** can be broken to allow the riboflavin solution to drain into the product bag/s. The product bag(s) can then be illuminated.

By having the photosensitizer already in the product bags or in a second bag preconnected to the product bags, the steps of connecting an illumination bag to each product bag, connecting a bag containing the photochemical to the illumination bag, flowing the product into the illumination bag, and flowing the photochemical solution into the product in the illumination bag are all eliminated, reducing the likelihood of contamination during the multiple connections.

An additional bag (not shown) containing the photosensitizer may also be attached to the separation bag **200.** The photosensitizer may be metered from the additional bag through the separation bag **200** into the product bags after the separated blood components have been collected in the product bags. Alternatively, the photosensitizer could be metered into the separation bag before the separation process. The separated blood products would already contain the photosensitizer when flowed into the product bags.

In a method of using the disposable separation and pathogen inactivation sets of the present invention, once the separated blood products have been directed from the separation vessel into their respective product bags containing photosensitizer, the bag(s) may be sterilely removed and sealed off from the rest of the bag set using any known methods.

If an opaque overwrap is part of the tubing set, once the separated blood components have been collected in the product bags, the removable opaque overwrap is removed from the product bags immediately before the blood component is illuminated.

The overwrap is not always necessary, for example when the photosensitizer is activated only by light not usually found in the environment in which the set will be used, for example ultraviolet or infra-red radiation.

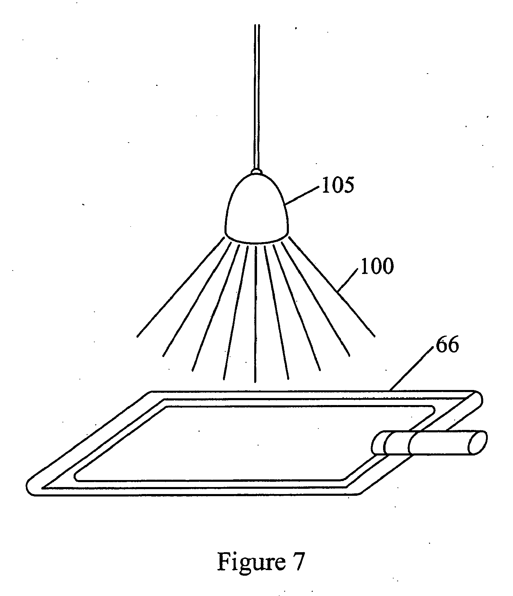

As shown in FIG. 7, after the bag (designated as element **66** for example purposes only) containing the platelet component to be inactivated is removed from the rest of the disposable bag set **70** (not shown and given as but one example), the opaque overwrap **77** is removed, and the bag is immediately exposed to radiation **100** from a light source **105** for a time sufficient to inactivate any pathogens which may be present in the blood product, but less than an amount which would cause damage to the blood product.

If the photosensitizer is added to the product bag from the bag containing photosensitizer, there would be no need for any opaque overwrap to be removed from the product bag.

The pathogen inactivated blood product may then be stored for a period of time before being transfused into a recipient, or may be transfused into a recipient immediately after the pathogen inactivation procedure.

It is understood for the purposes of this disclosure that various changes and modifications may be made to the invention that are well within the scope of the invention. Numerous other changes may be made which will readily suggest themselves to those skilled in the art and which are encompassed by the amended claims.

## Claims

1. A pre-connected disposable set for blood separation and pathogen reduction comprising:
a separation vessel;
a product collection conduit fluidly connected at one end to the separation vessel;
a first blood product bag fluidly connected to the other end of the product collection conduit; and
a photosensitizer contained in the first blood product bag.

2. The pre-connected disposable set of claim 1 further comprising a removable opaque overwrap covering the first blood product bag.

3. A pre-connected disposable set for blood separation and pathogen reduction comprising:
a separation vessel;
a product collection conduit fluidly connected at one end to the separation vessel;
at least one blood product bag fluidly connected to the other end of the product collection conduit;
a photosensitiser bag containing a photosensitiser; and
a conduit fluidly connecting the photosensitiser bag to the at least one blood product bag.

4. The pre-connected disposable set of claim 3, further comprising a removable opaque overwrap covering the photosensitiser bag.

5. The pre-connected disposable set of any one of the preceding claims, wherein the first blood product bag is a red blood cell product bag.

6. The pre-connected disposable set of any one of the preceding claims, wherein the first blood product bag is a platelet product bag.

7. The pre-connected disposable set of any one of the preceding claims, wherein the first blood product bag is a plasma product bag.

8. The pre-connected disposable set of claim 5, further comprising a platelet product bag.

9. The pre-connected disposable set of claim 5, 6 or 8, further comprising a plasma product bag.

10. The pre-connected disposable set of any one of the preceding claims, wherein the photosensitizer is a psoralen.

11. The pre-connected disposable set of any one of claims 1 to 9, wherein the photosensitizer is an endogenous alloxazine.

12. The pre-connected disposable set of any one of the preceding claims, further comprising:
a collection bag; and
a transfer tubing fluidly connecting the collection bag to the separation vessel.

13. A method of pathogen inactivating blood components which may contain pathogens comprising:
providing a first separated blood component;
flowing the first separated blood component into a pre-connected product bag containing a photosensitizer;
wherein the product bag is covered by a removable opaque overwrap;
removing the removable opaque overwrap; and
illuminating the first separated blood component and photosensitizer for a time sufficient to inactivate any pathogens which may be present in the blood component but less than a time which would cause damage to the blood component.

14. A method of pathogen inactivating blood components which may contain pathogens comprising:
providing a first separated blood component;
flowing the first separated blood component into a pre-connected product bag;
flowing a photosensitizer from a bag pre-connected to the product bag into the product bag; and
illuminating the product bag for a time sufficient to inactivate any pathogens which may be present in the blood component but less than a time which would cause damage to the blood component.

15. The method of claim 14, further comprising the step of removing the bag which had contained the photosensitizer from the product bag before the illuminating step.

16. The method of any one of claims 13 to 15, further comprising the step of initially separating whole blood in a separation vessel into at least said first blood component and wherein said first separated blood component is flowed from the separation vessel into the product bag pre-connected to said separation vessel.

17. The method of claim 16, further comprising the step of removing the product bag from the separation vessel before the illuminating step.
